(19) Europäisches Patentamt

European Patent Office

Office européen des brevets

(11) **EP 0 986 370 B1**

(12) **FASCICULE DE BREVET EUROPEEN**

(45) Date de publication et mention
de la délivrance du brevet:
**08.09.2004 Bulletin 2004/37**

(51) Int Cl.[7]: **A61K 7/48**, A61K 7/06,
A61K 35/78

(21) Numéro de dépôt: **98929508.4**

(22) Date de dépôt: **05.06.1998**

(86) Numéro de dépôt international:
**PCT/FR1998/001159**

(87) Numéro de publication internationale:
**WO 1998/055087 (10.12.1998 Gazette 1998/49)**

(54) **PRODUIT COSMETIQUE COMPRENANT DES EXTRAITS DE PLANTES**

KOSMETISCHE MITTEL ENTHALTENDE PFLANZENEXTRAKTEN

COSMETIC PRODUCT CONTAINING PLANT EXTRACTS

(84) Etats contractants désignés:
**AT BE CH CY DE DK ES FI FR GB GR IE IT LI LU
MC NL PT SE**

(30) Priorité: **06.06.1997 FR 9707207**

(43) Date de publication de la demande:
**22.03.2000 Bulletin 2000/12**

(73) Titulaire: **Cognis France S.A.
31360 Saint Martory (FR)**

(72) Inventeurs:
• **PAULY, Gilles
F-54000 Nancy (FR)**
• **FLEURY, Marie
F-75005 Paris (FR)**

(74) Mandataire: **Nuss, Pierre et al
Cabinet Nuss
10, rue Jacques Kablé
67080 Strasbourg Cédex (FR)**

(56) Documents cités:
• **STN, Serveur de bases de données,
XP002052904**
• **STN, Serveur de bases de données,
XP002052905**
• **STN, Serveur de bases de données,
XP002052906**
• **STN, Serveur de bases de données,
XP002052907**

## Description

La présente invention concerne le domaine de la cosmétique, plus particulièrement la dermo-cosmétique, et a pour objet l'utilisation d'extraits de plantes, présentes notamment dans le Haut-Maroni (Guyane Française), pour la préparation de produits cosmétiques pour la peau, les muqueuses et/ou les phanères.

Les femmes Aluku (Haut-Maroni - Guyane Française) effectuent traditionnellement, tous les matins et tous les soirs, une toilette intime rituelle avec une décoction végétale spécifique à chaque période de leur vie de femme et faisant appel à plusieurs dizaines de plantes locales.

Ces dernières sont préparées en décoction, puis sont utilisées en bain de siège en fonction de la période du cycle menstruel et de l'effet recherché.

L'étude de ces différentes plantes a fait apparaître certaines propriétés pharmaceutiques spécifiques, à savoir des propriétés antiseptiques, cicatrisantes, astringentes et toniques (voir "Végétaux utilisés pour l'hygiène intime des femmes Aluku en Guyane Française : interprétation culturelle et intérêt pharmacologique", FLEURY Marie, Actes du 2ème Colloque Européen d'Ethnopharmacologie et de la 11ème Conférence internationale d'Ethnomédecine, Heidelberg, 24-27 mars 1993).

Or, les inventeurs de la présente invention ont constaté, de manière inattendue et surprenante que des extraits de certaines des plantes visées ci-dessus présentaient, outre les propriétés pharmacologiques précitées, simultanément des propriétés et activités anti-radicaux libres, anti-vieillissement et stimulante de l'auto-synthèse de glutathion réduit, prononcées, sinon supérieures, du moins d'un niveau au moins équivalent à celles des actifs habituellement utilisés en cosmétique.

Ainsi, le principal objet de la présente invention consiste en l'utilisation, en tant qu'agent actif pour la préparation d'un produit cosmétique à usage topique pour la peau, les muqueuses et/ou les phanères d'au moins un extrait ou mélange d'extraits d'une plante choisie dans le groupe formé par Maprounea guianensis, Walteria indica, Gouania blanchetiana, Cordia schomburgkii, Randia armata et Hibiscus furcellatus, présentant une activité anti-radicalaire et stimulante de l'auto-synthèse de glutathion réduit renforcée.

Cette activité peut en particulier être avantageusement utilisée dans des préparations cosmétiques anti-vieillissement, anti-stress physique (UV-R), froid, chaleur, vent et anti-stress chimique (notamment pollution) et photoprotectrices ainsi que les préparations capillaires anti-stress et photoprotectrices ou encore les produits solaires ou après-soleil.

Les extraits précités peuvent, par exemple, être préparés à partir de la ou des plantes entière(s).

Toutefois, de manière avantageuse, lesdits extraits de plante(s) sont obtenus à partir des parties aériennes de ces dernières, par exemple les tiges, les feuilles, les fleurs et/ou les bourgeons.

Après collecte, les plantes ou parties de plantes concernées sont, éventuellement après séchage, soumises à un procédé d'extraction, préférentiellement en deux opérations successives aboutissant à deux extraits distincts, le solvant d'extraction mis en oeuvre pouvant être avantageusement choisi dans le groupe formé par l'eau, les solutions aqueuses à différents pH, les alcools en C1 - C6, les cétones (acétone, méthylcétone, di-éthylcétone), les hydrocarbures halogénés, les esters (acétate d'éthyle, de propyle, de butyle ou analogues), les polyols (glycols, diéthylèneglycol, propane diol, dipropylène glycol, butylène glycol) ou les mélanges d'au moins deux des substances précitées.

Selon un mode de réalisation particulier de l'invention, le ou les extrait(s) de plante(s) consiste(nt) en une ou des fraction(s) isolée(s) purifiée(s) extraite(s) d'une ou de plusieurs desdites plantes.

En vue d'aboutir à une composition cosmétique présentant, en outre, une activité anti-élastase renforcée, protectrice et réparatrice des fibres élastiques du derme, supérieure aux agents actifs anti-vieillissement actuellement utilisés en cosmétique, l'agent actif incorporé est avantageusement constitué par au moins un extrait de plante choisie dans le groupe formé par Maprounea guianensis, Gouania blanchetiana, Walteria indica et Cordia schomburgkii.

En vue d'aboutir à une composition cosmétique présentant en plus une activité anticollagènase importante, l'agent actif incorporé est avantageusement constitué par au moins un extrait de plante choisie dans le groupe formé par Maprounea guianensis, Walteria indica, et Randia armata.

En vue d'aboutir à une composition cosmétique induisant également un effet anti-UVA important, l'agent actif incorporé est avantageusement constitué par au moins un extrait de plante choisie dans le groupe formé par Maprounea guianensis et Walteria indica.

En vue d'aboutir à une composition cosmétique présentant en plus un effet anti-UVB important, l'agent actif incorporé est avantageusement constitué par au moins un extrait de plante choisie dans le groupe formé par Maprounea guianensis, Walteria indica, Gouania blanchetiana, et Randia armata.

En vue d'aboutir à une composition cosmétique présentant de plus une activité anti-tyrosinase renforcée induisant un effet dépigmentant supérieure aux actifs dépigmentants actuels, l'agent actif incorporé est avantageusement constitué par au moins un extrait de plante choisie dans le groupe formé par Maprounea guianensis et Gouania blanchetiana.

En vue d'aboutir à une composition cosmétique présentant en plus une forte activité stimulatrice du métabolisme cellulaire, l'agent actif incorporé est avantageusement constitué par au moins un extrait de plante choisie dans le groupe formé par Maprounea guianensis, Gouania blanchetiana, Cordia schomburgkii, et Hibiscus furcellatus.

En vue d'aboutir à une composition cosmétique présentant en plus une activité anti-glycation renforcée, l'agent actif incorporé est avantageusement constitué par au moins un extrait de plante choisie dans le groupe formé par Maprounea guianensis, Gouania blanchetiana, et Cordia schomburgkii.

Conformément à un mode de réalisation préférentiel de l'invention permettant d'aboutir à un agent actif présentant simultanément une activité anti-tyrosinase élevée, une très forte activité anti-protéases (anti-élastase et anti-collagènase), une très forte activité anti-UVA et anti-UVB, une action stimulatrice importante du métabolisme cellulaire et un effet anti-glycation important, il peut être prévu que l'agent actif soit préférentiellement consitué par un extrait obtenu à partir de la plante Maprounea guianensis.

A titre illustratif et non limitatif, on décrira ci-après différents procédés préférentiellement mis en oeuvre pour la réalisation des extraits de plantes mentionnés ci-dessus :

Ainsi, un procédé de préparation d'un extrait aqueux peut, par exemple, comprendre les étapes suivantes :

- mettre en suspension, dans un réacteur, la matière végétale broyée dans 5 à 10 volumes d'eau distillée ;
- extraire sous agitation pendant une heure à 85 - 90° C ;
- refroidir à température ambiante ;
- centrifuger pendant 15 min à 5 000 g ou filtrer grossièrement ;
- clarifier si besoin sur filtres en profondeur jusqu'à 0,5 µm ;
- noter le volume d'extrait E1 recueilli et déterminer sa teneur en matière sèche ;
- extraire et traiter le culot humide dans les mêmes conditions pour obtenir un extrait E2 ;
- déshydrater les deux extraits par atomisation de l'extrait végétal, éventuellement après addition d'un adjuvant tel la maltodextrine (2/3 d'adjuvant pour 1/3 de matière extraite). [Température d'entrée : 185 - 190° C / Température de sortie : 75 - 80° C.]

Un procédé de préparation d'un extrait hydroalcoolique peut, par exemple, comprendre les étapes suivantes :

- mettre en suspension, dans un réacteur, la matière végétale broyée dans 5 à 10 volumes d'éthanol aqueux ;
- extraire sous agitation pendant une heure à reflux ;
- filtrer sur un büchner muni d'un filtre en profondeur ;
- recueillir le surnageant, évaporer la phase éthanol sous pression réduite, centrifuger si nécessaire pendant 10 min à 5 000 g pour éliminer l'insoluble et filtrer ;
- extraire et traiter le culot humide dans les mêmes conditions afin d'obtenir un extrait E2 ;
- déshydrater les deux extraits par atomisation directe de l'extrait végétal, soit après addition d'un adjuvant tel la maltodextrine (2/3 d'adjuvant pour 1/3 de matière extraite).

Un procédé de préparation d'un extrait alcoolique peut, par exemple, comprendre les étapes suivantes :

- mettre en suspension, dans un réacteur, la matière végétale broyée dans 5 à 10 volumes d'éthanol ;
- extraire sous agitation pendant une heure à reflux ;
- refroidir à température ambiante ;
- filtrer sur un büchner muni d'un filtre en profondeur ;
- évaporer l'alcool sous pression réduite à 45° C ;
- sécher à l'étuve à 40° C ;
- extraire et traiter le culot humide dans les mêmes conditions pour obtenir un extrait E2.

En mettant en oeuvre les procédés de préparation décrits ci-dessus, différents extraits ont été obtenus à partir des plantes concernées et de Spondias mombin tels que mentionnés dans les exemples 1 à 7 ci-après.

Exemple 1 : des feuilles sèches de Spondias mombin ont été traitées selon les modes opératoires précédemment décrits afin d'obtenir des extraits aqueux, alcooliques et hydroalcooliques.

Les résultats suivants ont été obtenus :

| | Extrait (caractéristiques) | aqueux | hydro-alcoolique (éthanol 50 %) | éthanolique |
|---|---|---|---|---|
| | Poids de matière traitée (g) | 200 | 200 | 200 |

(suite)

|  | Extrait (caractéristiques) | aqueux | hydro-alcoolique (éthanol 50 %) | éthanolique |
|---|---|---|---|---|
| Premier extrait E1 | Couleur | brun foncé | brun | vert |
|  | Rendement d'extraction (%) | 17,12 | 23,00 |  |
|  | Poids sec d'extrait après séchage (g) | 26,02 | 33,90 | 37,35 |
|  | Rendement réel après séchage (%) | 13,01 | 16,95 | 18,68 |
| Deuxième extrait E2 | Couleur | brun | brun | brun foncé |
|  | Rendement d'extraction (%) | 7,29 | 7,60 |  |
|  | Poids sec d'extrait après séchage (g) | 8,14 | 9,23 | 9,38 |
|  | Rendement réel après séchage (%) | 4,07 | 4,62 | 4,69 |
| Rendement total (%) |  | 17,08 | 21,57 | 23,37 |

On notera que le rendement d'extraction correspond à la matière extraite présente dans la solution, c'est-à-dire

$$\frac{(\text{volume d'extrait x teneur en matière sèche}) \times 100}{\text{poids de matière de départ}}$$

et que le rendement réel après séchage correspond au rendement de matière obtenu sous forme sèche et tient compte des pertes au cours du procédé de séchage.

Exemple 2 : des feuilles sèches de Maprounea guianensis ont été traitées selon les modes opératoires précédemment décrits.

Les résultats suivants ont été obtenus :

|  | Extrait (caractéristiques) | aqueux | hydro-alcoolique (éthanol 50 %) | éthanolique |
|---|---|---|---|---|
|  | Poids de matière traitée (g) | 200 | 200 | 200 |
| Premier extrait (E1) | Couleur | brun-orangé | brun | brun |
|  | Rendement d'extraction (%) | 6,87 | nd |  |
|  | Poids sec d'extrait après séchage (g) | 7,00 | 36,65 | 24,67 |
|  | Rendement réel après séchage (%) | 3,50 | 18.33 | 12,34 |
| Deuxième extrait (E2) | Couleur | brun-orangé | brun | brun foncé |
|  | Rendement d'extraction (%) | 13,25 | nd |  |
|  | Poids sec d'extrait après séchage (g) | 15,04 | 25,14 | 13,30 |
|  | Rendement réel après séchage (%) | 7,52 | 12,57 | 6,65 |
| Rendement total (%) |  | 11,02 | 30,90 | 18,99 |

Exemple 3 : des feuilles sèches de Gouania blanchetiana ont été traitées selon les modes opératoires précédemment décrits.

Les résultats suivants ont été obtenus :

| | Extrait (caractéristiques) | aqueux | hydro-alcoolique (éthanol 50 %) | éthanolique |
|---|---|---|---|---|
| | Poids de matière traitée (g) | 200 | 200 | 200 |
| Premier extrait (E1) | Couleur | brun | brun | vert |
| | Rendement d'extraction (%) | 6,10 | 11,86 | |
| | Poids sec d'extrait après séchage (g) | 8,90 | 13,28 | 13,87 |
| | Rendement réel après séchage (%) | 4,45 | 6,64 | 6,94 |
| Deuxième extrait (E2) | Couleur | brun | brun | brun foncé |
| | Rendement d'extraction (%) | 5,05 | 5,00 | |
| | Poids sec d'extrait après séchage (g) | 5,07 | 4,67 | 5,22 |
| | Rendement réel après séchage (%) | 2,54 | 2,34 | 2,61 |
| **Rendement total (%)** | | **6,99** | **8,98** | **9,55** |

Exemple 4 : des feuilles sèches de Cordia schomburgkii ont été traitées selon les modes opératoires précédemment décrits afin d'obtenir des alcooliques et hydroalcooliques.

Dans cet exemple, les extraits E1 et E2 de l'extrait alcoolique ont été rassemblés avant séchage (pour l'extraction hydroalcoolique, un adjuvant est ajouté avant atomisation : les poids correspondants sont les poids de matière extraite et ne tiennent pas compte du poids d'adjuvant).

Les résultats suivants ont été obtenus :

| | Extrait (caractéristiques) | hydro-alcoolique (éthanol 70 %) | éthanolique |
|---|---|---|---|
| | Poids de matière traitée (g) | 200 | 200 |
| Premier extrait (E1) | Couleur | vert | vert |
| | Rendement d'extraction (%) | 14,60 | |
| | Poids sec d'extrait après séchage (g) | 20,81 | 17,16 |
| | Rendement réel après séchage (%) | 10,41 | 8,58 |
| Deuxième extrait (E2) | Couleur | | vert |
| | Rendement d'extraction (%) | | |
| | Poids sec d'extrait après séchage (g) | | 10,13 |
| | Rendement réel après séchage (%) | | 5,07 |
| **Rendement total (%)** | | | **13,65** |

Exemple 5 : un mélange de feuilles et de bourgeons secs de Walteria indica a été traité selon les modes opératoires précédemment décrits afin d'obtenir des extraits aqueux, alcooliques et hydroalcooliques.

Les résultats suivants ont été obtenus :

| | Extrait (caractéristiques) | aqueux | hydro-alcoolique (éthanol 50 %) | éthanolique |
|---|---|---|---|---|
| | Poids de matière traitée (g) | 150 | 150 | 150 |

(suite)

| | Extrait (caractéristiques) | aqueux | hydro-alcoolique (éthanol 50 %) | éthanolique |
|---|---|---|---|---|
| Premier extrait (E1) | Couleur | brun | brun | vert |
| | Rendement d'extraction (%) | 9,83 | 9,82 | |
| | Poids sec d'extrait après séchage (g) | 7,27 | 7,05 | 10,31 |
| | Rendement réel après séchage (%) | 4,85 | 4,70 | 6,87 |
| Deuxième extrait (E2) | Couleur | brun | brun | vert |
| | Rendement d'extraction (%) | 5,05 | 1,70 | |
| | Poids sec d'extrait après séchage (g) | 4,03 | 2,16 | 3,06 |
| | Rendement réel après séchage (%) | 2,69 | 1,44 | 2,04 |
| Rendement total (%) | | 7,53 | 6,14 | 8,91 |

Exemple 6 : des feuilles sèches de Randia armata ont été traitées selon les modes opératoires précédemment décrits afin d'obtenir des extraits aqueux, alcooliques et hydroalcooliques.

Dans cet exemple, les extraits E1 et E2 sont rassemblés avant séchage (pour les extractions aqueuse et hydroalcoolique, un adjuvant est ajouté avant atomisation : les poids correspondants sont les poids de matière extraite et ne tiennent pas compte du poids d'adjuvant).

Les résultats suivants ont été obtenus :

| | Extrait (caractéristiques) | aqueux | hydro-alcoolique (éthanol 50 %) | éthanolique |
|---|---|---|---|---|
| | Poids de matière traitée (g) | 200 | 200 | 200 |
| Premier extrait (E1) | Couleur | brun clair | brun | vert |
| | Rendement d'extraction (%) | 25,50 | 36,50 | |
| | Poids sec d'extrait après séchage (g) | 34,00 | 59,75 | 51,38 |
| | Rendement réel après séchage (%) | 17,00 | 29,88 | 25,69 |
| Deuxième extrait (E2) | Couleur | | | vert |
| | Rendement d'extraction (%) | | | |
| | Poids sec d'extrait après séchage (g) | . | | 15,15 |
| | Rendement réel après séchage (%) | | | 7,58 |
| Rendement total (%) | | | | 33,27 |

Exemple 7 : des tiges feuillées sèches d'Hibiscus furcellatus ont été traitées selon les modes opératoires précédemment décrits afin d'obtenir des extraits alcooliques et hydroalcooliques.

Dans cet exemple, les extraits E1 et E2 de l'extrait hydroalcoolique sont rassemblés avant séchage (pour l'extraction hydroalcoolique, un adjuvant est ajouté avant atomisation : les poids correspondants sont les poids de matière extraite et ne tiennent pas compte du poids d'adjuvant).

Les résultats suivants ont été obtenus :

| | Extrait (caractéristiques) | hydro-alcoolique (éthanol 50 %) | éthanolique |
|---|---|---|---|
| | Poids de matière traitée (g) | 150 | 150 |

EP 0 986 370 B1

(suite)

|  | Extrait (caractéristiques) | hydro-alcoolique (éthanol 50 %) | éthanolique |
|---|---|---|---|
| **Premier extrait (E1)** | Couleur | brun-vert | vert |
|  | Rendement d'extraction (%) | 8,99 | |
|  | Poids sec d'extrait après séchage (g) | 7,96 | 3,78 |
|  | Rendement réel après séchage (%) | 5,31 | 2,52 |
| **Deuxième extrait (E2)** | Couleur | | brun foncé |
|  | Rendement d'extraction (%) | | |
|  | Poids sec d'extrait après séchage (g) | | 1,22 |
|  | Rendement réel après séchage (%) | | 0,81 |
| **Rendement total (%)** | | | **3,33** |

Les propriétés et activités cosmétiquement intéressantes des extraits de plantes précités, découvertes par les inventeurs, ont pu être mises en évidence et être évaluées quantitativement par l'intermédiaire de différents tests connus de l'homme du métier qui sont explicités succintement ci-après et dont les résultats ont été rassemblés dans le tableau I.

1) <u>Mise en évidence d'une activité dépigmentante</u>

Afin de déterminer le pouvoir dépigmentant des extraits, leur activité tyrosinase a été évaluée par dosage du produit d'oxydation de la L-DOPA en DOPACHROME (selon la technique de Mason HS, 1948) en effectuant les étapes suivantes :

- mélanger à la pipette automatique, la tyrosinase avec la L-DOPA dans le tampon phosphate,
- enregistrer la densité optique à 475 nm durant 2 minutes puis,
- calculer de la vitesse initiale de la réaction enzymatique et,
- déterminer la CI50 (concentration qui réduit de moitié la vitesse initiale de formation de DOPACHROME).

En se reportant au tableau I ci-après, on notera que les résultats montrent de façon surprenante et inattendue un net effet anti-tyrosinase des extraits de plantes comparable, voire supérieur, à la substance de référence (hydro-quinone).

2) <u>Mise en évidence d'une activité anti-radicalaire</u>

L'agression des radicaux libres constitue l'un des mécanismes majeurs de l'endommagement cutané suite aux actions des facteurs de stress du milieu extérieur.

Les radicaux libres, notamment les formes réactives de l'oxygène (anion superoxyde $O_2^{-\bullet}$, radical hydroxyl $HO^\bullet$, oxygène singulet $O_2^l$, peroxyde d'hydrogène $H_2O_2$) agissent à l'échelon cellulaire : notamment endommagement des lipides membranaires avec formation de lipoperoxydes, altération des protéines, blocage des systèmes enzymatiques et altération du capital génétique nucléaire. Des effets délétères se produisent également au niveau des macromolécules de la matrice extracellulaire du derme, à savoir au niveau du collagène, de l'élastine et des glycoaminoglycanes.

De façon également surprenante et inattendue, il a été observé une activité antiradicalaire puissante des différents extraits obtenus (voir tableau I ci-après).

Cette activité a été' évaluée par les tests chimiques et tests biochimiques in tubo suivants :

a) <u>Activité antiradicaux libres</u> : épreuve au DPPH (Deby C, 1970)

Le DPPH (diphénylpicrylhydrazyl) est un radical stable qui, dissous dans un méthanol, forme une solution colorée en violet.

Une substance à activité antiradicalaire stabilise le DPPH en un leucodérivé (incolore).

b) <u>Activité anti HO$^\bullet$</u> : méthode à l'acide salicylique (Tien M, 1982)

Le fer complexé par l'acide éthylènediaminetétracétique forme en solution aqueuse des radicaux hydroxyles HO$^\bullet$ au contact d'$H_2O_2$.

Les radicaux hydroxyles réagissent avec l'aide salicylique (A.S.) pour former un composé coloré en rougeâtre. Une substance à activité antiradicalaire absorbe ces radicaux HO$^\bullet$ et réduit ainsi la formation de composé coloré en rouge.

c) <u>Activité anti HO$^\bullet$</u> : méthode au désoxyribose (méthode de Halliwell, 1987 ou méthode de Aruoma, 1988).

Les radicaux hydroxyles HO$^\bullet$ sont produits par la réaction de Fenton :

$$Fe^{++} + H_2O_2 \rightarrow Fe^{+++} + HO^\bullet + OH^-.$$

Le fer est complexé avec l'EDTA dans la méthode de Halliwell et n'est pas complexé dans la méthode de Aruoma, afin d'évaluer les capacités de capture par la substance à évaluer (exemple : la desferioxamine inactive la réaction de Fenton par capture du fer).

Les radicaux hydroxyles sont révélés par un sucre, le desoxyribose (présent dans l'ADN) qui forme, en présence d'HO$^\bullet$, un composé réagissant avec l'acide thiobarbiturique (TBA) sous forme d'un condensat rosé et fluorescent.

d) <u>Activité anti $O_2^-{}^\bullet$</u> : méthode au luminol (Oyamburo GM, 1970)

Un système enzymatique (hypoxanthine/xanthine oxydase : XOD) forme des anions superoxydes radicalaires $O_2^-{}^\bullet$, qui réagissent avec le luminol pour former un composé intermédiaire qui, en se stabilisant, émet une luminescence enregistrée à l'aide d'un photomultiplicateur.

Une substance à activité antiradicalaire absorbe ou détruit l'anion $O_2^-{}^\bullet$ et ainsi réduit la formation de luminescence.

e) <u>Activité anti $O_2^-{}^\bullet$ et $H_2O_2$</u> : méthode au luminol + microperoxydase (Israël M, 1985)

Un système enzymatique (hypoxanthine + xanthine oxydase : XOD) forme des anions superoxydes radicalaires $O_2^-{}^\bullet$ qui se dismutent lentement en $H_2O_2$ et $O_2$.

$H_2O_2$ et $O_2^-{}^\bullet$ réagissent avec la microperoxydase (M) pour former $O_2^l$ (oxygène singulet) qui dégrade le luminol (Lu) pour former un composé luminescent, dont l'émission de lumière est enregistrée à l'aide d'un photomultiplicateur.

Une substance antiradicalaire absorbe soit $H_2O_2$, soit $O_2^-{}^\bullet$ ou encore $O_2^l$ et réduit ainsi la formation de luminescence.

f) <u>Activité anti $O_2^-{}^\bullet$</u> : méthode au neotetrazolium bleu (NBT) (Ohkuma N, 1987)

Un système enzymatique (hypoxanthine / xanthine oxydase) forme des anions superoxydes radicalaires $O_2^-{}^\bullet$

Les anions $O_2^-{}^\bullet$ réagissent avec le NBT pour former un composé rouge : le formazan.

Une substance antiradicalaire absorbe ou détruit l'anion $O_2^-{}^\bullet$ et réduit ainsi la formation de formazan.

3) <u>Mise en évidence d'une activité anti-UV-A</u>

Le rayonnement UV-A est un facteur déterminant du vieillissement cutané, soit par altération de la matrice dermique, soit par endommagement cellulaire.

De façon surprenante et inattendue, il a été observé une nette activité protectrice cellulaire contre les UV-A des différents extraits de plantes obtenus.

Des fibroblastes humains du type MRC5 ont été mis en culture dans un milieu de croissance jusqu'à saturation du tapis cellulaire.

Le milieu de croissance a ensuite été remplacé par un milieu standard (DMEM + SVF à 1 %) contenant chaque extrait aux diverses doses à tester (voir tableau I).

Après incubation pendant 48 heures à 37° C, les différents milieux ont été remplacés par une solution saline (Hanks) puis les MRC5 ont été irradiés par dessus, avec des tubes fluorescents.

Dès la fin de l'irradiation, la solution saline a été prélevée pour le dosage du malonaldialdehyde (MDA) par réaction avec l'acide thiobarbiturique à chaud (fluorescence à 560 nm). Le MDA est un produit de dégradation des lipides insaturés qui composent les membranes biologiques. Il provoque des pontages qui inhibent les enzymes et forment des lipofuscines (taches de vieillesse) et serait mutagène.

Les cellules MRC5 ont été récupérées dans la soude diluée pour le dosage des protéines (méthode de Bradford) et du glutathion (GSH) par une sonde fluorescente (Hissin, 1976). Le GSH apporte au cytoplasme un potentiel réducteur nécessaire au bon fonctionnement de la machinerie cellulaire ; par ailleurs le GSH capte les formes réactives de l'oxygène et permet grâce à la GSH-peroxydase la régénération des lipides peroxydés.

4) Mise en évidence de l'effet cytoprotecteur anti-UVB sur kérotinocytes humains en survie "in vitro"

Les UVB déclenchent une inflammation (érythème, oedème) par activation d'une enzyme la phospholipase A2.

Cette enzyme décroche l'acide arachidonique des phospholipides de la membrane plasmique : l'acide arachidonique est le précurseur des prostaglandines qui sont des médiateurs de l'inflammation et ainsi les prostaglandines E2 (= PGE2) sont formées par la cyclooxygènase.

Les kératinocytes A431 sont ensemencés dans un milieu défini avec du sérum de veau foetal..

Après 72 heures à 37° C et $CO_2$ = 5 %, le milieu de culture est remplacé par une solution saline contenant l'extrait végétal à tester.

Les kératinocytes sont aussitôt irradiés par une dose d'UVB (30mJ/$cm^2$ ; tubes du type DUKE FL40E) incubés 24 heures à 37° C, puis les taux de PGE2 et de LDH sont mesurés dans le milieu surnageant.

Le nombre de kératinocytes adhérents est déterminé (après trypsination) par un compteur de particules et le taux de PGE2 est déterminé par un test ELISA.

On détermine également par une réaction enzymatique le taux de LDH (lactate-deshydrogénase / enzyme cytoplasmique), qui constitue un marqueur de la souffrance cellulaire.

L'activité de l'extrait végétal testé est exprimé en % de variation des deux marqueurs par rapport aux valeurs obtenues avec les témoins (moyenne de 2 essais, chacun en duplicate - voir tableau I).

5) Mise en évidence d'une activité anti-vieillissement

L'élastine est une macromolécule dermique organisée en réseau structuré donnant à la peau ses propriétés d'élasticité.

Ce réseau élastique se dégrade avec l'âge, l'endommagement s'aggravant sur les parties découvertes exposées au rayonnement solaire et le résultat est une perte d'élasticité et une flaccidité importante de la peau.

L'un des mécanismes de ce processus est l'hyperactivité enzymatique de digestion des fibres élastiques par les élastases endogènes et l'effet bénéfique des inhibiteurs d'élastase en tant que substance active anti-vieillissement est bien connu.

De façon surprenante et inattendue, il a été observé une nette activité anti-élastase des extraits obtenus (cf tableau I).

La méthode utilisée pour révéler l'inhibition de l'élastase est la méthode avec substrat synthétique, qui comporte principalement les étapes suivantes :

- utilisation de N-succinyl-(Ala.)3-para-Nitroanilide à 1 mM dans un tampon TRIS (pH = 8) ;
- incubation de la substance avec l'élastase et ce substrat durant 20 minutes à 20° C ;
- apparition d'une coloration jaune, dosée par spectrophotométrie à 410 nm.

6) Mise en évidence d'une activité anti-collagènase in tubo (selon Van Wart en 1981)

Les protéases sécrétées par les polymorphonucléaires neutrophiles lors de l'inflammation ou par les fibroblastes soumis à une irradiation par les UVA, provoquent une dégradation des protéines qui structurent la matrice extracellulaire du derme.

L'activité anti-collagénase in tubo d'un extrait est mesurée avec une collagénase de clostridium hystoliticium et un substrat synthétique chromogène : le FALGPA.

L'incubation est de 30 mn à température ambiante et la densité optique est mesurée à 324 nm.

Les résultats sont exprimés en % d'inhibition de l'enzyme par rapport au témoin sans actif (Tableau I).

7) Mise en évidence d'une activité inhibitrice de la glycation non-enzymatique in tubo (activité "anti-glycation" selon Devi en 1990 et Monnier en 1984).

La glycation non-enzymatique des protéines est un processus déterminant du viellissement des tissus humains et explique la réticulation des matrices extracellulaires et des membranes basales, responsable en grande partie des pathologies observées chez les diabétiques.

De plus, les bases de Schiff catalysent la production de formes réactives de l'oxygène qui aggravent les effets de la glycation non-enzymatique.

Les tests in tubo ont été réalisés sur du collagène type I incubé 21 jours à 45° C en présence de glucose à 1 % et le taux de bases de Shiff est évalué par densitométrie à 350 nm et fluorimétrie à 430 nm (excitation à 350 nm).

Les résultats sont exprimés en % d'inhibition de la formation de base de Schiff par rapport aux témoins (Tableau I).

### 8) Mise en évidence de la stimulation du métabolisme cellulaire

L'activité stimulante du métabolisme amène à une augmentation de la consommation d'oxygène. Les actifs ayant cette propriété participent à une meilleur régénération cellulaire et peuvent être utilisés avec succès dans le traitement des peaux fatiguées, stressées, exposées à la pollution ainsi que pour combattre l'effet du viellissement.

La consommation d'oxygène est déterminée par des mesures polarographiques sur un homogénat de cellules épithéliales en utilisant un oxygraphe au type de celui fabriqué par la société Gilson, muni d'une électrode dite de Clark.

L'enregistrement de la consommation d'oxygène est effectué d'abord dans les conditions témoin (cellules épithéliales dans un tampon) et ensuite après addition de la substance à tester dans le milieu, la concentration finale de la substance à tester étant de 1 %.

La consommation d'oxygène est calculée d'après les courbes polarographiques et l'effet du produit est exprimé en pourcentage d'augmentation de la consommation d'oxygène par rapport aux conditions témoin (en absence de l'actif à tester).

Les résultats correspondent à la moyenne +/- SEM de 3 essais (Tableau I).

Comme le montre le tableau I ci-après, qui réserve les résultats des 8 tests décrits ci-dessus, les extraits de plantes obtenus présentent également tous, de manière prononcée, une activité anti-radicalaire et stimulante de l'auto-synthèse de glutathion réduit, à usage pour les préparations cosmétiques anti-vieillissement, les préparations cosmétiques anti-stress physiques (UV-R, froid, thermique, vent), anti-stress chimiques (par exemple pollution).

# Tableau I

| Extrait | Plante | Effet anti-tyrosinase CI50 en % | Test DPPH | A.S. | Desoxyribose | Lu. | Lu. + M | NBT | MDA | GSH | PGE2 | LDH | Inhibition en % (Effet anti-élastase à 0,3 %) | Inhibition en % (Effet anti-collagénase à 0,3 %) | Effet anti-glycation % d'inhibition de formation de bases de Schiff / doses 0,01 à 0,1 % | Stimulation du métabolisme % d'augmentation de la consommation d'O₂ (m+/-SEM) doses = 1 % |
|---|---|---|---|---|---|---|---|---|---|---|---|---|---|---|---|---|
| Extrait éthanolique exemple 1 | Spondias Mombin | 0,017 | 92 | 40 | 46 | 71 | 100 | 97 | 68 | -61 | 30 | -73 | -88 | 87 | 99 | 43 | 93 +/- 24 |
| Extrait aqueux exemple 2 | Maprounea Guianensis | 0,015 | 92 | 35 | 55 | 76 | 100 | 99 | 74 | -34 | 38 | -99 | -100 | 80 | 8 | 100 | 63 +/- 7 |
| Extrait éthanolique exemple 2 | Maprounea Guianensis | 0,015 | 91 | 47 | 32 | 79 | 100 | 99 | 70 | -53 | 45 | -100 | -100 | 85 | 100 | | 61 +/- 20 |
| Extrait éthanol 50 % exemple 3 | Gouania Blanchetiana | 0,015 | 88 | 0 | 27 | 67 | 100 | 91 | 75 | -13 | 14 | -92 | -100 | 56 | 39 | 68 | 151 +/- 48 |
| Extrait aqueux exemple 5 | Walteria Indica | 0,08 | 88 | 16 | 8 | 47 | 100 | 96 | 62 | -43 | 71 | -25 | -70 | 48 | 83 | 3 | 57 +/- 29 |
| Extrait éthanol 70 % exemple 4 | Cordia Shomburgkii | 0,09 | 89 | 30 | 3 | 19 | 99 | 97 | 31 | -25 | 10 | | | 37 | 17 | 54 | 79 +/- 17 |
| Extrait aqueux exemple 6 | Randia Armata | 0,18 | 92 | 12 | 0 | 34 | 100 | 96 | 40 | -15 | 37 | 20 | -70 | 16 | 97 | 11 | - 54 |
| Extrait éthanol 50 % exemple 6 | Randia Armata | 0,13 | 92 | 18 | 0 | 42 | 100 | 96 | 38 | -11 | 43 | -70 | -89 | 17 | 99 | | - 41 |
| Extrait éthanol 50 % exemple 7 | Hibiscus Furcellatus | 0,83 | 86 | 19 | 0 | 14 | 99 | 42 | 10 | 0 | 42 | -2 | -17 | 17 | 13 | 0 | 118 +/- 31 |
| | Hydro-quinone | 0,025 | | | | | | | | | | | | | | | |

La présente invention a également pour objet une composition ou un produit cosmétique à usage topique externe pour la peau, les muqueuses et/ou les phanères comportant à titre d'agent(s) actif(s), présentant notamment une activité anti-radicalaire et stimulante de l'auto-synthèse de glutathion réduit renforcée, seul(s) ou associé(s) à au moins un autre agent actif, au moins un extrait d'une plante choisie dans le groupe formé par Maprounea guianensis, Walteria indica, Gouania blanchetiana, Cordia schomburgkii, Randia armata et Hibiscus furcellatus.

Selon un premier mode de réalisation préférentiel de l'invention, et en vue d'obtenir une composition cosmétique présentant cumulativement, en plus des propriétés avantageuses indiquées ci-dessus, une activité anti-tyrosinase, une activité anti-élastase, une activité anti-UVB et une activité anti-glycation importantes, l'agent actif est constitué par au moins un extrait de plante choisie dans le groupe formé par Maprounea guianensis et Gouania blanchetiana.

Conformément à un second mode de réalisation préférentiel de l'invention, et en vue d'obtenir une composition cosmétique présentant cumulativement, en plus des propriétés avantageuses indiquées ci-dessus, une activité anti-protéases renforcée (anti-élastase et anti-collagènase) ainsi que des effets anti-UVA et anti-UVB importants, l'agent actif est constitué par au moins un extrait de plante choisie dans le groupe formé par Maprounea guianensis et Walteria indica.

De manière avantageuse, la composition cosmétique contient, en tant qu'agent actif, unique ou associé à d'autres agents actifs, entre 0,001 % et 20 % en poids, préférentiellement entre 0,1 % et 3 % en poids, d'un extrait ou d'un mélange d'extraits de plante(s), tel(s) que défini(s) ci-dessus.

A titre d'exemples non limitatifs de réalisations pratiques de l'invention, on décrira ci-après différents produits ou préparations cosmétiques comprenant un extrait de plante ou un mélange d'extraits de plantes tel(s) que décrit(s) précédemment.

Exemple 1 :

Un produit cosmétique sous forme de crème blanchissante pour la peau conforme à l'invention pourra, par exemple, présenter une composition pondérale, constituée à partir des fractions A, B et C suivantes, telle qu'indiquée ci-après.

Fraction A :

- Stéarate de glycérol (et) Cétéareth 20       15,00 %
- Huile de paraffine      3,00 %
- Palmitate d'ascorbyle      3,00 %
- Diméthicone      3,00 %
- Alcool cétylique      0,50 %
- PEG 30 - Isostéarate de glycérol      2,00 %

Fraction B :

- Eau      72,20 %
- Méthylparaben      0,20 %
- Imidazolidinyl urée      0,30 %
- Extrait éthanolique de Gouania blanchetiana (selon exemple 3)      0,50 %

Fraction C :

- Parfum      0,30 %

Le procédé de préparation et de fabrication de la crème susvisée consiste à faire fondre les ingrédients de la fraction A sous agitation à 75° C, à préparer la fraction B à 75° C, puis à verser la fraction A dans la fraction B sous agitation turbine, à laisser refroidir sous agitation planétaire et à ajouter la fraction C.

Exemple 2 :

Un produit cosmétique sous forme d'émulsion anti-taches pour les mains conforme à l'invention pourra, par exemple, présenter une composition pondérale, constituée à partir des fractions A, B et C suivantes, telle qu'indiquée ci-après.

Cette émulsion pourra également constituer, conformément à l'invention, un produit multi-actif notamment anti-radicalaire, anti-UVA et anti-UVB, anti-protéases, anti-glycation et stimulant du métabolisme cellulaire.

Fraction A :

- Stéarate de glycérol (et) Stéarate PEG 100        6,00 %
- Alcool oléïque        1,50 %
- Stéarate de glycérol        2,00 %
- Stéareth-2        2,00 %
- Beurre de karité        3,00 %
- Diméthicone        4,00 %
- Triglycéride caprylique / caprique        8,00 %
- Propylparaben        0,10 %
- Acétate de tocophérol        0,10 %

Fraction B :

- Eau        62,30 %
- Elestab 388
  . (Laboratoires Sérobiologiques)        2,50 %
- Extrait aqueux de Maprounea
  guianensis (selon exemple 2)        1,00 %
- Extrait éthanolique de Spondias
  mombin (selon exemple 1)        0,50 %
- Propylène glycol        5,00 %

Fraction C :

- Polyacrylamide (et) isoparaffine (et) Laureth 7        2,00 %

Le procédé de préparation et de fabrication de l'émulsion susvisée consiste à préparer séparément les fractions A et B à 75° C, à ajouter la fraction A dans la fraction B sous agitation turbine à 75° C, puis à refroidir le mélange obtenu à 50° C et à ajouter la fraction C et enfin à refroidir le mélange final à température ambiante.

Exemple 3 :

Un produit cosmétique sous forme de conditionneur anti-stress et photoprotecteur pour cheveux secs conforme à l'invention pourra par exemple, présenter une composition pondérale, constituée à partir des fractions A et B suivantes, telle qu'indiquée ci-après.

Fraction A :

- Alcool cétylique        2,00 %
- Huile de paraffine        2,00 %
- Stéarate de sorbitol        1,00 %

- Palmitate d'isopropyle (et) huile de ricin     1,00 %

Fraction B :

- Glycérine     2,00 %
- Laureth 20     1,00 %
- Chlorure de cétrimonium     2,00 %
- Extrait éthanolique de Spondias mombin (selon exemple 1)     0,50 %
- Extrait aqueux de Randia armata     0,10 %
- Elestab 388
  (Laboratoires Serobiologiques)     1,50 %
- Eau     q.s.p. 100,00 %

Le procédé de préparation et de fabrication du conditionneur susvisé consiste à préparer séparément les fractions A et B sous agitation à 80° C, à ajouter la fraction A dans la fraction B sous agitation turbine et enfin à refroidir le mélange obtenu à température ambiante.

Exemple 4 :

Un produit cosmétique sous forme de crème protectrice et anti-stress pour le corps conforme à l'invention pourra, par exemple, présenter une composition pondérale, constituée à partir des fractions A, B et C suivantes, telle qu'indiquée ci-après.

Fraction A :

- Stéarate de glycérol (et) Cétéareth 20 (et) Cétéareth 10 (et) alcool cétostéarylique (et) palmitate cétylique     6,00 %
- Alcool cétostéarylique     1,00 %
- Oléate de décyle     3,00 %
- Huile de paraffine     4,00 %
- Beurre de karité     2,00 %

Fraction B :

- Glycérine     3,00 %
- Protéines de froment hydrolysées     0,50 %
- Extrait éthanolique d'Hibiscus furcellatus (selon exemple 7)     1,25 %
- Extrait aqueux de Randia armata (selon exemple 6)     0,60 %
- Extrait éthanolique de Cordia schomburgkii (selon exemple 4)     0,20 %
- Eau     78,25 %

Fraction C :

- Parfum     0,20 %

Le procédé de préparation et de fabrication de l'émulsion susvisée consiste à préparer séparément les fractions A et B sous agitation à 80° C, puis à ajouter la fraction A dans la fraction B sous agitation turbine, à ramener ensuite le mélange obtenu à température ambiante et à ajouter la fraction C.

Exemple 5 :

Un produit cosmétique sous forme de crème de jour anti-vieillissement et multi-active (anti-radicalaire, anti-UVA et anti-élastase) conforme à l'invention pourra, par exemple, présenter une composition pondérale, constituée à partir des fractions A, B et C suivantes, telle qu'indiquée ci-après.

Fraction A :

- Stéarate de glycérol        14,00 %
- Octyldodécanol        6,00 %
- Adipate de dibutyle        6,00 %
- Cétéareth 12        1,50 %
- Cétéareth 20        1,50 %

Fraction B :

- Propylène glycol        5,00 %
- Extrait éthanolique de Maprounea guianensis
  (selon exemple 2)        0,75 %
- Extrait aqueux de Walteria indica
  (selon exemple 5)        1,00 %
- Extrait éthanolique de Randia armata
  (selon exemple 6)        0,50 %
- Elestab 4112
  (Laboratoires Serobiologiques)        0,40 %
- Eau        q.s.p. 100,00 %

Fraction C :

- Parfum        0,20 %

Le procédé de préparation et de fabrication de la crème susvisée consiste à préparer séparément les fractions A et B sous agitation à 80° C, puis à ajouter la fraction A dans la fraction B sous agitation turbine, puis à refroidir le mélange obtenu à 45° C, à ajouter ensuite la fraction C et enfin à ramener le mélange final à température ambiante.
Bien entendu, l'invention n'est pas limitée aux modes de réalisation décrits. Des modifications restent possibles, notamment du point de vue de la constitution des divers éléments ou par substitution d'équivalents techniques, sans sortir pour autant du domaine de protection de l'invention.

**Revendications**

1. Utilisation, en tant qu'agent actif pour la préparation d'un produit cosmétique à usage topique pour la peau, les muqueuses et/ou les phanères, présentant des activités anti-radicalaire et stimulante de l'auto-synthèse de glutathion réduit renforcées, d'au moins un extrait d'une plante choisie dans le groupe formé par Maprounea guianensis, Waltheria indica, Gouania blanchetiana, Cordia schomburgkii, Randia armata et Hibiscus furcellatus.

2. Utilisation selon la revendication 1, **caractérisée en ce que** le ou les extraits de plante(s) est (sont) obtenu(s) à partir des parties aériennes de ces dernières.

3. Utilisation selon l'une quelconque des revendications 1 et 2, **caractérisée en ce que** le solvant mis en oeuvre pour la réalisation des extractions est choisi dans le groupe formé par l'eau, les solutions aqueuses à différents pH, les alcools en C1 - C6, les cétones, les hydrocarbures halogénés, les esters, les polyols et les mélanges d'au moins deux des substances précitées.

**4.** Utilisation selon l'une quelconque des revendications 1 à 3, **caractérisée en ce que** le ou les extrait(s) de plante (s) consiste(nt) en une ou des fraction(s) isolée(s) purifiée(s) extraite(s) d'une ou de plusieurs desdites plantes.

**5.** Utilisation selon l'une quelconque des revendications 1 à 4, **caractérisée en ce que** l'agent actif, présentant de plus une activité anti-élastase renforcée, est constitué par au moins un extrait de plante choisie dans le groupe formé par Maprounea guianensis, Gouania blanchetiana, Waltheria indica et Cordia schomburgkii.

**6.** Utilisation selon l'une quelconque des revendications 1 à 4, **caractérisée en ce que** l'agent actif, présentant en plus une activité anti-collagènase renforcée, est constitué par au moins un extrait de plante choisie dans le groupe formé par Maprounea guianensis, Waltheria indica et Randia armata.

**7.** Utilisation selon l'une quelconque des revendications 1 à 4, **caractérisée en ce que** l'agent actif, induisant de plus un effet anti-UVA important, est constitué par au moins un extrait de plante choisie dans le groupe formé par Maprounea guianensis et Waltheria indica.

**8.** Utilisation selon l'une quelconque des revendications 1 à 4, **caractérisée en ce que** l'agent actif, présentant en plus un effet anti-UVB important, est constitué par au moins un extrait de plante choisie dans le groupe formé par Maprounea guianensis, Waltheria indica, Gouania blanchetiana et Randia armata.

**9.** Utilisation selon l'une quelconque des revendications 1 à 4, **caractérisée en ce que** l'agent actif, présentant de plus une activité anti-tyrosinase renforcée, est constitué par au moins un extrait de plante choisie dans le groupe formé par Maprounea guianensis et Gouania blanchetiana.

**10.** Utilisation selon l'une quelconque des revendications 1 à 4, **caractérisée en ce que** l'agent actif, présentant en plus une forte activité stimulatrice du métabolisme cellulaire, est constitué par au moins un extrait de plante choisie dans le groupe formé par Maprounea guianensis Gouania blanchetiana, Cordia schomburgkii et Hibiscus furcellatus.

**11.** Utilisation selon l'une quelconque des revendications 1 à 4, **caractérisée en ce que** l'agent actif, présentant en plus une activité anti-glycation renforcée, est constitué par au moins un extrait de plante choisie dans le groupe formé par Maprounea guianensis, Gouania blanchetiana et Cordia schomburgkii.

**12.** Utilisation selon l'une quelconque des revendications 1 à 11, **caractérisée en ce que** l'agent actif est constitué par un extrait obtenu à partir de la plante Maprounea guianensis.

**13.** Composition cosmétique à usage topique pour la peau, les muqueuses ou les phanères, **caractérisée en ce qu'**elle comporte à titre d'agent actif présentant des activités anti-radicalaire et stimulante de l'auto-synthèse de glutathion réduit renforcées, seul ou associé à au moins un autre agent actif, au moins un extrait d'une plante choisie dans le groupe formé par Maprounea guianensis, Waltheria indica, Gouania blanchetiana, Cordia schomburgkii, Randia armata et Hibiscus furcellatus.

**14.** Composition cosmétique selon la revendication 13, **caractérisée en ce qu'**elle comporte un agent actif présentant notamment une activité anti-tyrosinase, une activité anti-élastase, une activité anti-UVB et une activité anti-glycation importantes et constitué par au moins un extrait de plante choisie dans le groupe formé par Maprounea guianensis et Gouania blanchetiana.

**15.** Composition cosmétique selon la revendication 13, **caractérisée en ce qu'**elle comporte un agent actif, présentant une activité anti-protéases renforcée, notamment anti-élastase et anti-collagènase, ainsi que des effets anti-UVA et anti-UVB importants et constitué par au moins un extrait de plante choisie dans le groupe formé par Maprounea guianensis et Waltheria indica.

**16.** Composition cosmétique à usage topique pour la peau, présentant notamment une activité anti-radicalaire et stimulante de l'auto-synthèse de glutathion réduit renforcée, **caractérisée en ce qu'**elle contient, en tant qu'agent actif, unique ou associé à d'autres agents actifs, entre 0,001 % et 20 % en poids, préférentiellement entre 0,1 % et 3 % en poids, d'un extrait ou d'un mélange d'extraits de plante(s) selon l'une quelconque des revendications 13 à 15.

**Patentansprüche**

1. Verwendung von mindestens einem Extrakt einer Pflanze aus der Gruppe Maprounea guianensis, Waltheria indica, Gouania blanchetiana, Cordia schomburgkii, Randia armata und Hibiscus furcellatus als Wirkstoff zur Herstellung eines Kosmetikprodukts zur topischen Verwendung für die Haut, die Schleimhäute und/oder die Körperanhanggebilde, der insbesondere eine verstärkte Wirkung gegen Radikale und eine verstärkte, die Autosynthese von reduziertem Glutathion fördernde Wirkung aufweist.

2. Verwendung nach Anspruch 1, **dadurch gekennzeichnet, daß** der Pflanzenextrakt bzw. die Pflanzenextrakte aus den oberirdischen Teilen der Pflanzen gewonnen werden.

3. Verwendung nach einem der Ansprüche 1 und 2, **dadurch gekennzeichnet, daß** das zur Extraktion verwendete Lösungsmittel aus der Gruppe Wasser, wäßrige Lösungen mit unterschiedlichen pH-Werten, C1-C6-Alkohole, Ketone, Halogenkohlenwasserstoffe, Ester, Polyole und Mischungen aus mindestens zwei der genannten Stoffe gewählt ist.

4. Verwendung nach einem der Ansprüche 1 bis 3, **dadurch gekennzeichnet, daß** der Pflanzenextrakt bzw. die Pflanzenextrakte aus einer gereinigten isolierten Fraktion, die aus einer oder mehreren dieser Pflanzen extrahiert wurde, bzw. aus mehreren gereinigten isolierten Fraktionen, die aus einer oder mehreren dieser Pflanzen extrahiert wurden, besteht bzw. bestehen.

5. Verwendung nach einem der Ansprüche 1 bis 4, **dadurch gekennzeichnet, daß** der Wirkstoff, der außerdem eine verstärkte Wirkung gegen Elastase aufweist, aus mindestens einem Pflanzenextrakt aus der Gruppe Maprounea guianensis, Gouania blanchetiana, Waltheria indica und Cordia schomburgkii besteht.

6. Verwendung nach einem der Ansprüche 1 bis 4, **dadurch gekennzeichnet, daß** der Wirkstoff, der außerdem eine verstärkte Wirkung gegen Kollagenase aufweist, aus mindestens einem Pflanzenextrakt aus der Gruppe Maprounea guianensis, Waltheria indica und Randia armata besteht.

7. Verwendung nach einem der Ansprüche 1 bis 4, **dadurch gekennzeichnet, daß** der Wirkstoff, der außerdem zu einer beträchtlichen Wirkung gegen UVA führt, aus mindestens einem Pflanzenextrakt aus der Gruppe Maprounea guianensis und Waltheria indica besteht.

8. Verwendung nach einem der Ansprüche 1 bis 4, **dadurch gekennzeichnet, daß** der Wirkstoff, der außerdem eine starke Wirkung gegen UVB aufweist, aus mindestens einem Pflanzenextrakt aus der Gruppe Maprounea guianensis, Waltheria indica, Gouania blanchetiana und Randia armata besteht.

9. Verwendung nach einem der Ansprüche 1 bis 4, **dadurch gekennzeichnet, daß** der Wirkstoff, der außerdem eine verstärkte Wirkung gegen Tyrosinase aufweist, aus mindestens einem Pflanzenextrakt aus der Gruppe Maprounea guianensis und Gouania blanchetiana besteht.

10. Verwendung nach einem der Ansprüche 1 bis 4, **dadurch gekennzeichnet, daß** der Wirkstoff, der außerdem eine stark stimulierende Wirksamkeit auf den Zellmetabolismus aufweist, aus mindestens einem Pflanzenextrakt aus der Gruppe Maprounea guianensis, Gouania blanchetiana, Cordia schomburgkii und Hibiscus furcellatus besteht.

11. Verwendung nach einem der Ansprüche 1 bis 4, **dadurch gekennzeichnet, daß** der Wirkstoff, der außerdem eine verstärkte Wirksamkeit gegen Glycierung aufweist, aus mindestens einem Pflanzenextrakt aus der Gruppe Maprounea guianensis, Gouania blanchetiana und Cordia schomburgkii besteht.

12. Verwendung nach einem der Ansprüche 1 bis 11, **dadurch gekennzeichnet, daß** der Wirkstoff aus einem Extrakt der Pflanze Maprounea guianensis. besteht.

13. Kosmetikzusammensetzung zur topischen Verwendung für die Haut, die Schleimhäute oder die Körperanhanggebilde, **dadurch gekennzeichnet, daß** sie als Wirkstoff, der insbesondere eine Wirkung gegen Radikale und eine die verstärkte Autosynthese von reduziertem Glutathion stimulierende Wirkung aufweist, allein oder zusammen mit anderen Wirkstoffen, mindestens einen Pflanzenextrakt aus der Gruppe Maprounea guianensis, Waltheria indica, Gouania blanchetiana, Cordia schomburgkii, Randia armata und Hibiscus furcellatus enthält.

**14.** Kosmetikzusammensetzung nach Anspruch 13, **dadurch gekennzeichnet, daß** sie mindestens einen Wirkstoff aufweist, der insbesondere eine beträchtliche Wirkung gegen Tyrosinase, Elastase, UVB und Glycierung aufweist und aus mindestens einem Pflanzenextrakt aus der Gruppe Maprounea guianensis und Gouania blanchetiana besteht.

**15.** Kosmetikzusammensetzung nach Anspruch 13, **dadurch gekennzeichnet, daß** sie einen Wirkstoff enthält, der eine verstärkte Wirkung gegen Proteasen, insbesondere Elastase und Kollagenase, sowie beträchtliche Wirkungen gegen UVA und UVB aufweist und aus mindestens einem Pflanzenextrakt aus der Gruppe Maprounea guianensis und Waltheria indica besteht.

**16.** Kosmetikzusammensetzung zur topischen Verwendung für die Haut, die insbesondere eine Wirkung gegen Radikale und eine die verstärkte Autosynthese von reduziertem Glutathion stimulierende Wirkung aufweist, **dadurch gekennzeichnet, daß** sie als Wirkstoff allein oder zusammen mit anderen Wirkstoffen zwischen 0,001 Gew.-% und 20 Gew.-%, vorzugsweise zwischen 0,1 Gew.-% und 3 Gew.-%, eines Pflanzenextrakts oder einer Pflanzenextraktmischung nach einem der Ansprüche 13 bis 15 enthält.


**Claims**

**1.** Use, as an active agent for preparing a cosmetic product for topical application to the skin, the mucous membranes and/or superficial body growths, having increased anti-radical activity and increased activity in stimulating reduced glutathione auto-synthesis, of at least one extract of a plant selected from the group formed by Maprounea guianesis, Waltheria indica, Gouania blanchetiana, Cordia schmoburgkii, Randia armata and Hibiscus furcellatus.

**2.** Use according to claim 1, **characterised in that** the plant extract or extracts is/are obtained from aerial parts of said plants.

**3.** Use according to any one of claims 1 and 2, **characterised in that** the solvent used for carrying out the extractions is selected from the group formed by water, aqueous solutions with different pH levels, C1 - C6 alcohols, ketones, halogenated hydrocarbons, esters, polyols, and mixtures of at least two of the aforementioned substances.

**4.** Use according to any one of claims 1 to 3, **characterised in that** the plant extract or extracts consists/consist of one or more purified isolated fractions extracted from one or more of said plants.

**5.** Use according to any one of claims 1 to 4, **characterised in that** the active agent, which also has increased anti-elastase activity, consists of at least one plant extract selected from the group formed by Maprounea guianensis, Gouania blanchetiana, Waltheria indica and Cordia schomburgkii.

**6.** Use according to any one of claims 1 to 4, **characterised in that** the active agent, which also has increased anti-collagenase activity, consists of at least one plant extract selected from the group formed by Maprounea guianesis, Waltheria indica and Randia armata.

**7.** Use according to any one of claims 1 to 4, **characterised in that** the active agent, which also produces a significant anti-UVA effect, consists of at least one plant extract selected from the group formed by Maprounea guianesis and Waltheria indica.

**8.** Use according to any one of claims 1 to 4, **characterised in that** the active agent, which also has a significant anti-UVB effect, consists of at least one plant extract selected from the group formed by Maprounea guianesis, Waltheria indica, Gouania blanchetiana and Randia armata.

**9.** Use according to any one of claims 1 to 4, **characterised in that** the active agent, which also has increased anti-tyrosinase activity, consists of at least one plant extract selected from the group formed by Maprounea guianesis and Gouania blanchetiana.

**10.** Use according to any one of claims 1 to 4, **characterised in that** the active agent, which also has a strong cellular metabolism-stimulating activity, consists of at least one plant extract selected from the group formed by Maprounea guianesis, Gouania blanchetiana, Cordia schomburgkii and Hibiscus furcellatus.

11. Use according to any one of claims 1 to 4, **characterised in that** the active agent, which also has increased anti-glycation activity, consists of at least one plant extract selected from the group formed by Maprounea guianensis, Gouania blanchetiana and Cordia schomburgkii.

12. Use according to any one of claims 1 to 11, **characterised in that** the active agent consists of an extract obtained from the plant Maprounea guianensis.

13. Cosmetic composition for topical application to the skin, the mucous membranes and/or superficial body growths, **characterised in that** it comprises as an active agent having increased anti-radical activity and increased activity in stimulating reduced glutathione auto-synthesis, on its own or combined with at least one other active agent, at least one extract of a plant selected from the group formed by Maprounea guianesis, Waltheria indica, Gouania blanchetiana, Cordia schomburgkii, Randia armata and Hibiscus furcellatus.

14. Cosmetic composition according to claim 13, **characterised in that** it comprises an active agent having, in particular, significant anti-tyrosinase, anti-elastase, anti-UVB and anti-glycation activities and consisting of at least one plant extract selected from the group formed by Maprounea guianesis and Gouania blanchetiana.

15. Cosmetic composition according to claim 13, **characterised in that** it comprises an active agent having increased anti-protease, in particular anti-elastase and anti-collagenase, activity, as well as significant anti-UVA and anti-UVB effects, and consisting of at least one plant extract selected from the group formed by Maprounea guianesis and Waltherica indica.

16. Cosmetic composition for topical application to the skin, having, in particular, increased anti-radical activity and increased activity in stimulating reduced glutathione auto-synthesis, **characterised in that** it contains as an active agent, on its own or combined with other active agents, between 0.001% and 20% by weight, preferably between 0.1 % and 3% by weight, of a plant extract or of a mixture of plant extracts according to any one of claims 13 to 15.